# EUROPEAN PATENT SPECIFICATION

(11) **EP 3 998 984 B1**
(45) Date of publication and mention of the grant of the patent: **12.04.2023**
(21) Application number: 20740036.7
(22) Date of filing: 17.07.2020
(51) Int. Cl.: A61C 5/64, A61J 1/06, A61M 5/24, A61M 5/28, A61M 5/31

(54) **CARTRIDGE FOR DISPENSING A MATERIAL**
KARTUSCHE ZUR ABGABE EINES MATERIALS
CARTOUCHE POUR DISTRIBUER UN MATÉRIAU

(30) Priority: 19.07.2019 EP 19187180
(43) Date of publication of application: 25.05.2022
(73) Proprietor: Septodont ou Septodont SAS ou Specialites Septodont, 94100 Saint-Maur-des-Fossés (FR)
(72) Inventor: MARIE, Olivier, 94100 Saint-Maur-des-Fossés (FR); RICHARD, Gilles, 94100 Saint-Maur-des-Fossés (FR); CO, Clémence, 94100 Saint-Maur-des-Fossés (FR); ARTAUD, Laurent, 94100 Saint-Maur-des-Fossés (FR); CHABRIER, Olivier, 94100 Saint-Maur-des-Fossés (FR)
(74) Representative: Icosa
(86) International application number: PCT/EP2020/070358
(87) International publication number: WO 2021/013752

(56) References cited:
- WO-A1-2012/115022
- JP-A- 2001 104 339
- JP-A- 2010 142 408
- US-A1- 2003 103 409
- US-A1- 2006 178 638
- US-A1- 2011 224 640

## Description

### FIELD OF INVENTION

The present invention relates to a cartridge for dispensing a material. The material to be dispensed may be a one-component material or a multi-component system material, e.g. a two-component system material comprising a powder component and a liquid component. In the latter case, the cartridge of the invention is advantageously suitable both for the mixing and the dispensing of the material. A cartridge according to the invention may be used, in particular, for the dispensing of a dental material.

### BACKGROUND OF INVENTION

Cartridges suitable for the mixing and dispensing of material are particularly interesting for dental restoration applications, in order to mix a multi-component dental restoration material and dispense it on a tooth of a subject. A dental restoration material classically comprises a powder component and a liquid component, which are mixed to form a dental cement. The steps of weighing each component and mixing the two components are tedious, hazardous and time-consuming. To overcome this issue, cartridges have been developed for dental restoration applications, in which certain amounts of a powder component and of a liquid component are previously weighed and accommodated in isolated chambers so as to prevent any accidental mixing of the two components. In such cartridges, the components can be released from the isolated chambers at a desired time and mechanically mixed with each other to form a dental cement that is dispensed from the cartridge.

For example, US 6,386,872 B1 discloses a cartridge for dental restoration material, comprising successively a first tubular member, a second tubular member and a plunger located one inside the other, wherein the first tubular member contains a powder component and the second tubular member contains a liquid component, a dispensing nozzle being connected to the first tubular member. A first transverse wall separates the first tubular member and the second tubular member, whereas a second transverse wall separates the first tubular member and the nozzle. Upon applying a force on the plunger, an aperture is pierced in a rupturable portion of the first transverse wall, thus mixing the two components to form a dental restoration material. When it is moved further towards the nozzle, the plunger ruptures a rupturable portion of the second transverse wall, and the dental restoration material is dispensed from the nozzle. In such a cartridge, there is a risk that pieces of the rupturable portions migrate in the material during the dispensing, thus polluting the material. In addition, pieces of the rupturable portions may clog the nozzle and interfere with the dispensing of the material. Such a cartridge also has to be handled carefully in order to avoid uncontrolled dispensing of the material.

EP 2 692 308 A1 discloses a cartridge for the mixing and dispensing of material, comprising a body containing a main chamber, a dispensing nozzle, a liquid receptacle and a plunger having a forwardly projecting sharp protrusion. Upon depression of the plunger, the sharp protrusion ruptures a membrane of a front portion of the liquid receptacle, thus pushing hydraulically the liquid out of the liquid receptacle into the main chamber of the body to form a mixture with a powder contained in the main chamber. The front portion of the liquid receptacle is configured to be detached from the rest of the liquid receptacle by continued depression of the plunger, such that the plunger is able to traverse the entire length of the main chamber to rupture a membrane at the distal end of the main chamber and dispense the mixture from the main chamber into the dispensing nozzle. Such a cartridge comprising rupturable membranes that separate the liquid receptacle from the main chamber and the main chamber from the nozzle does not allow a fine control of hygrometry in each chamber. In the case of a powder highly sensitive to moisture, this limits the shelf life of the cartridge, since the powder in the main chamber is not properly protected from moisture. In addition, there is a risk that pieces of the ruptured membranes migrate in the material during the dispensing, thus polluting the material, or clog the nozzle and interfere with the dispensing of the material. Here again, the cartridge also has to be handled carefully to avoid uncontrolled dispensing of the material.

In the same way, problems of pollution of the material and clogging of the dispensing nozzle may arise in cartridges suitable for the dispensing of one-component materials, which are also subject to constraints in terms of sealing and maintaining a constant humidity level inside the chamber of the cartridge in the case of materials highly sensitive to moisture.

It is these drawbacks that the invention is intended more particularly to remedy by proposing a cartridge for dispensing a material which is easy to operate while making the dispensing of the material more secure, in particular by limiting the risks of polluting the material or clogging the dispensing nozzle, the cartridge also advantageously allowing a control of hygrometry in the cartridge, thus being compatible with long-term preservation of moisture sensitive materials. There is also a need for providing a cartridge for dispensing a material featuring good sealing properties, especially when delivering the material.

Documents WO 2012/115022 A1 and US 2006/178638 A1 can each be considered as closest prior art to the subject-matter of the appended independent claim 1.

### SUMMARY

For this purpose, a subject of the invention is a cartridge for dispensing a material, comprising:
- a sleeve having a longitudinal axis and comprising a distal wall provided with a dispensing hole;
- a barrel defining a chamber configured to receive the material, the barrel comprising a proximal open end and a breakable distal wall at its distal end, the barrel being configured to move in the sleeve along the longitudinal axis between a first position, in which the distal wall of the barrel is at a distance from the distal wall of the sleeve, and a second position, in which a sealing portion of the distal wall of the barrel is in contact with an inner surface of the distal wall of the sleeve;
- a piston configured to seal the chamber of the barrel, the piston comprising a plate configured to move in sealing engagement in the chamber of the barrel;
wherein the sleeve comprises an internal piercing element in the vicinity of the dispensing hole, the piercing element being configured to break the distal wall of the barrel upon transition of the barrel from the first position to the second position. According one embodiment, the piercing element is not or does not comprise a needle.

In a conventional manner, within the frame of the invention, the terms "proximal" and "distal" refer to a position of portions of the cartridge with reference to an operator (e.g. a dentist), i.e. the term "proximal" refers to a position closer to the operator of the cartridge, while the term "distal" refers to a position that is more distant from the operator.

In the cartridge of the invention, the distal wall of the barrel is broken by means of the internal piercing element when the barrel moves from the first position to the second position, which corresponds to a transition of the cartridge to a dispensing configuration. Thanks to the specific arrangement of the piercing element in the vicinity of the dispensing hole, the force applied by the piercing element on the distal wall of the barrel is oriented in a direction away from a dispensing nozzle connected to the dispensing hole, so that the broken parts of the distal wall of the barrel are pushed away from the nozzle. In this way, the risks of polluting the material to be dispensed or clogging the nozzle are efficiently reduced. The cartridge of the invention also allows a control of hygrometry in the chamber of the barrel, which is hermetically sealed by the piston, thus being compatible with the storage of moisture sensitive materials. Examples of moisture sensitive materials that are to be kept in a controlled environment with low hygrometry include, in a non-limitative manner: powder components of multi-component dental restoration materials, intended to be stored separately and mixed extemporaneously with a liquid; one-component materials such as calcium sulphate-based cements or calcium silicate-based cements.

According to one embodiment, the piercing element is a tubular element projecting internally from the distal wall of the sleeve, said piercing element being in fluid communication with the dispensing hole. Thanks to this arrangement, once the distal wall of the barrel has been broken, the tubular piercing element forms a privileged passageway guiding the material from the barrel directly to the dispensing hole, which limits the losses of material in the interspace between the barrel and the sleeve.

According to one embodiment, the inner diameter of the piercing element is higher than or equal to the diameter of the dispensing hole. In this way, the opening created in the distal wall of the barrel under the action of the piercing element has a size suitable to ensure proper flow of the material to be dispensed, which is particularly important for the dispensing of a pasty material.

According to one embodiment, the sleeve comprises an annular cavity around the piercing element, the annular cavity being delimited peripherally by a beveled inner surface complementary to a corresponding beveled outer surface of the barrel. The complementary beveled surfaces provide an efficient sealing at the interface between the barrel and the sleeve, close to the piercing element and the dispensing hole, which limits the passage of material in the interspace between the barrel and the sleeve, thus ensuring optimal dispensing control.

According to one embodiment, the distal wall of the barrel comprises a central breakable portion surrounded by the sealing portion, the ratio of the surface area of the breakable portion to the surface area of the sealing portion being such that, after opening, the fins of the open breakable portion leave the dispensing hole of the sleeve completely cleared, while the bearing surface of the sealing portion is sufficiently thin to limit the contact area. In this way, the seal is easier to achieve with the distal wall of the sleeve and the contact pressure is maximized to ensure sealing during the dispensing of the material. In particular, the relative dimensions of the breakable portion and the sealing portion are advantageously configured to ensure a proper flow of the material to be dispensed while limiting the material losses.

According to one embodiment, the distal wall of the barrel comprises at least one weakened portion having a thickness lower than the thickness of the rest of the distal wall. Preferably, the distal wall of the barrel comprises a plurality of weakened portions distributed on the breakable portion, so as to guide a specific deformation of the distal wall of the barrel when it is submitted to the action of the piercing element. In one embodiment, the weakened portions are weakened lines distributed around a central portion of the distal wall of the barrel, so as to guide a deformation of the distal wall in a corolla shape oriented away from a dispensing nozzle connected to the dispensing hole. In a particular embodiment, the weakened portions comprise weakened lines distributed radially starting from a central portion of the distal wall of the barrel, notably according to a star shape.

Advantageously, the distal wall of the barrel is broken under the action of the piercing element without detachment of pieces thereof that may pollute the material or clog the nozzle connected to the dispensing hole. More specifically, the distal wall of the barrel is configured so that the thrust force applied by the piercing element opens the distal wall at the weakened portions and deforms the portions of the distal wall surrounding the openings thus formed.

According to one embodiment, the barrel is made of a material having a Water Vapor Transfer Rate (WVTR) at 23°C and 85% relative humidity (RH), for a film thickness of 100 µm, of less than 0.5 g/(m²-day), preferably less than 0.4 g/(m²-day), such as a cyclic olefin copolymer (COC), a cyclic olefin polymer (COP), polypropylene (PP), high-density polyethylene (HDPE). A barrel made of such a low WVTR material makes it possible to maintain a constant humidity level in the chamber, in particular a humidity level suitable to protect a moisture sensitive material contained therein, such as a moisture sensitive powder component of a multi-component dental restoration material, or a moisture sensitive one-component material.

According to one embodiment, the barrel comprises at least one tab near its proximal end configured to cooperate with a corresponding housing of the sleeve to lock the barrel in the first position with respect to the sleeve. In this way, the cartridge comprises a locking system ensuring that the distal wall of the barrel can be broken only when the or each tab of the barrel is disengaged from the corresponding housing of the sleeve. Thus, in the locked configuration, the cartridge can be manipulated without any risk of undesired dispensing of the material, which is particularly useful when a mixing step is required prior to the dispensing step, as is the case for a cartridge intended for the dispensing of a multi-component system material.

According to one embodiment, the barrel comprises at least one outer radial feature configured to cooperate with at least one inner radial feature of the sleeve to keep the barrel in the first position with respect to the sleeve, even when the or each tab of the barrel is disengaged from the corresponding housing of the sleeve. Such cooperating radial features between the barrel and the sleeve make it possible to secure the cartridge in a storage configuration even when the locking system is not active. According to one embodiment, the barrel comprises at least two outer radial features configured to cooperate with at least two inner radial features of the sleeve to keep the barrel in the first position with respect to the sleeve.

According to one embodiment, the sleeve comprises at least one, two, three or four longitudinal groove(s) near the proximal end configured to guide the displacement of a tab of the barrel when the barrel is moved from the first position to the second position. The or each longitudinal groove guides the barrel towards the piercing element and improves the stability of the displacement of the barrel to reach the dispensing configuration of the cartridge.

According to one embodiment, the barrel comprises at least one, two, three or four inner radial feature(s) configured to cooperate with at least one, two, three or four outer radial feature(s) of the piston to keep the plate of the piston at a distance from the distal wall of the barrel before the piston is moved towards the distal wall of the barrel for the dispensing of the material. Such cooperating radial features between the barrel and the piston make it possible to have a two-step transition of the cartridge towards the dispensing configuration, obtained through the application of one and the same thrust force on the proximal end of the piston, i.e. including: a first step of initiating the dispensing of the material, in which the barrel is displaced relative to the sleeve from the first position to the second position, until the distal wall of the barrel is in contact with the distal wall of the sleeve and broken by the piercing element, the radial features between the barrel and the piston being mutually engaged in this first step so that the barrel and the piston move integrally with each other; and a second step of dispensing the material through the dispensing nozzle, in which the piston is displaced relative to the barrel, the radial features between the barrel and the piston being mutually disengaged in this second step. The transition from the first step to the second step happens automatically under the action of the thrust force, since the cooperation between the inner radial features of the barrel and the outer radial features of the piston is released once the distal wall of the barrel is in contact with the distal wall of the sleeve. According to one embodiment, the thrust force ranges from 50 N to more than 400 N, preferably from 50 N to 400 N, preferably from 200 N to 400 N.

According to one feature, the cartridge comprises a dispensing nozzle connected to the dispensing hole. The dispensing nozzle may be formed in one piece with the sleeve of the cartridge, or the dispensing nozzle may be attached to the sleeve of the cartridge by any appropriate means, for example by means of a Luer Lock connector. According to one embodiment, the cartridge comprises a deformable dispensing nozzle connected to the dispensing hole, wherein the thickness of the wall of the dispensing nozzle decreases from the dispensing hole toward a free end of the dispensing nozzle. According to one embodiment, the thickness of the wall of the dispensing nozzle ranges from 0.1 mm to 2 mm, preferably from 0.6 mm to 1.6 mm. Such a geometry of the dispensing nozzle, combined with the selection of an appropriate material for the dispensing nozzle, makes it possible to have an orientable nozzle having a constant internal diameter, even when it is bent. Examples of suitable materials for the dispensing nozzle include polyesters, polycarbonate, polyamide, polypropylene, high-density polyethylene. According to one embodiment, the suitable materials for the dispensing nozzle comprise or consists of polyesters, polycarbonate, polyamide, polypropylene, high-density polyethylene or copolymers thereof.

According to one embodiment, the piston comprises a distal rod configured to be received in an internal volume of the piercing element when the cartridge is in a configuration of end of distribution of the material. The distal rod configured to enter the tubular piercing element makes it possible to push the material to be dispensed as far as possible towards the dispensing hole and nozzle, thus limiting the losses of material.

According to one embodiment, the piston comprises a combination of a receptacle and a plunger, the receptacle defining a chamber and comprising a proximal open end and an openable distal wall, the plunger being movable in the chamber of the receptacle and comprising a distal rod configured to apply a pressure on the openable distal wall. A cartridge comprising such a two-part piston is suitable for the dispensing of a two-component material, in particular a material comprising a powder component initially hermetically received in the chamber of the barrel and a liquid component initially hermetically received in the chamber of the receptacle, the components being mixed with each other at a desired time to form the material to be dispensed, by opening the openable distal wall of the receptacle through application of a pressure thereon with the distal rod of the plunger.

According to one embodiment, the openable distal wall of the receptacle comprises a detachable portion attached to the rest of the distal wall by an annular connecting part having a solid portion extending over an angle of between 30° and 90°, preferably between 40° and 80°, more preferably of the order of 60°, and a weakened portion having a thickness less than the thickness of the solid portion, so as to form a hinge. This arrangement makes it possible to open the distal wall of the receptacle by rupturing only the weakened portion of the annular connecting part, whereas the detachable portion remains connected to the rest of the distal wall of the receptacle through the solid portion, which plays the role of a hinge. In this way, the detachable portion remains attached to the rest of the distal wall of the receptacle in the open configuration of the distal wall, i.e. the distal wall of the receptacle can be open under the action of the distal rod of the plunger without detachment of pieces that may pollute the material to be dispensed or clog the dispensing nozzle connected to the dispensing hole of the cartridge.

According to one embodiment, the receptacle comprises at least one inner radial feature configured to cooperate with at least one outer radial feature of the plunger to keep the plunger at a distance from the openable distal wall of the receptacle in a storage configuration of the cartridge, the cooperation between the inner radial feature and the outer radial feature of the plunger being releasable under the effect of a thrust force applied on the proximal end of the plunger. Such cooperating radial features between the receptacle and the plunger make it possible to secure the cartridge containing a two-component material in a storage configuration where the two components are separated, one being received in the chamber of the barrel while the other is received in the chamber of the receptacle, without risk of accidental mixing of the two components.

Another subject of the invention is a method of dispensing a material by means of a cartridge as described above, comprising steps in which:
- in a first step of initiating the dispensing of the material, under the action of a thrust force applied on the proximal end of the piston, the barrel is displaced relative to the sleeve from the first position to the second position, until the distal wall of the barrel is in contact with the distal wall of the sleeve and broken by the piercing element, the barrel and the piston being moved integrally with one another in this first step;
- in a second step of dispensing the material through the dispensing nozzle, under the action of a thrust force applied on the proximal end of the piston, the piston is displaced relative to the barrel.

### DESCRIPTION OF THE DRAWINGS

Features and advantages of the invention will become apparent from the following description of several embodiments of a cartridge according to the invention, this description being given merely by way of example and with reference to the appended drawings in which:
**Figure 1** is an exploded perspective view of a cartridge according to a first embodiment of the invention, suitable for mixing and dispensing a two-component system material comprising a powder component and a liquid component;
**Figure 2** is a longitudinal section of the cartridge of Figure 1, in an initial storage configuration of the cartridge;
**Figure 3** is a view in the direction of arrow III of Figure 2;
**Figure 4** is a longitudinal section similar to Figure 2, in a first activated configuration of the cartridge;
**Figure 5** is a longitudinal section similar to Figure 2, in a second activated configuration of the cartridge;
**Figure 6** is a view in the direction of arrow VI of Figure 5;
**Figure 7** is a longitudinal section similar to Figure 2, in a first dispensing configuration of the cartridge corresponding to the beginning of distribution of the material;
**Figure 8** is a longitudinal section similar to Figure 2, in a second dispensing configuration of the cartridge corresponding to the end of distribution of the material;
**Figure 9** is a longitudinal section of the sleeve of the cartridge of Figure 1;
**Figure 10** is a longitudinal section of the barrel of the cartridge of Figure 1;
**Figure 11** is a view in the direction of arrow XI of Figure 10;
**Figure 12** is a longitudinal section of the liquid receptacle of the cartridge of Figure 1;
**Figure 13** is a view in the direction of arrow XIII of Figure 12;
**Figure 14** is a longitudinal section of the plunger of the cartridge of Figure 1;
**Figure 15** is a longitudinal section similar to Figure 5 for a cartridge according to a second embodiment of the invention, suitable for dispensing a one-component material; and
**Figure 16** is a longitudinal section similar to Figure 5 for a cartridge according to a third embodiment of the invention, suitable for dispensing a one-component material.

### ILLUSTRATIVE EMBODIMENTS OF THE INVENTION

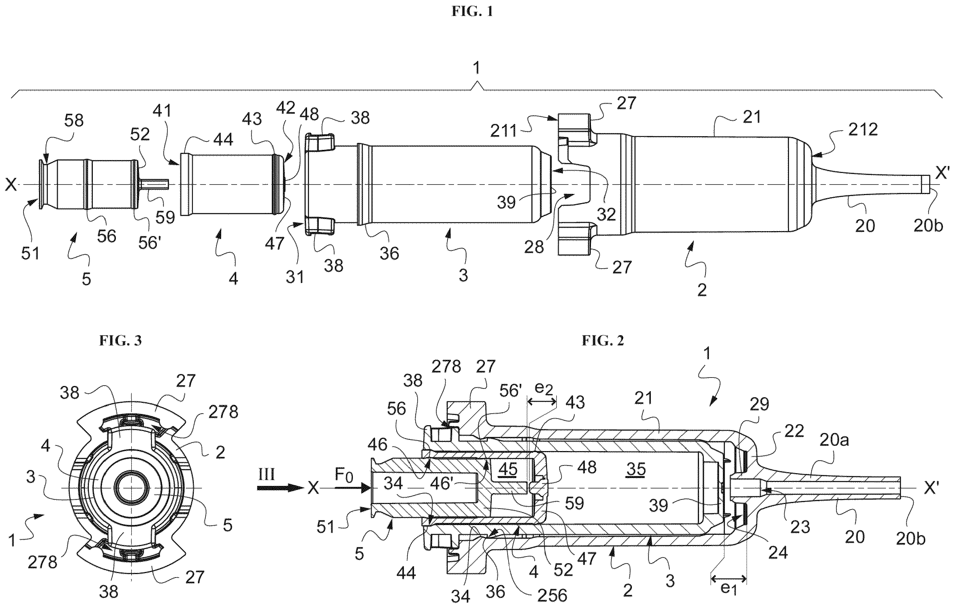

Figure 1 shows an exploded view of a cartridge 1 according to a first embodiment of the invention, suitable both for the mixing and the dispensing of a two-component dental restoration material comprising a powder component and a liquid component. As visible in Figure 1, the cartridge 1 comprises successive tubular members with circular cross sections, which are configured to be inserted one inside the other while being aligned along their longitudinal axis X-X'. In this first embodiment, the tubular members of the cartridge 1 include, going from the outermost element to the innermost element, an external sleeve 2, a barrel 3, a liquid receptacle 4 and a plunger 5.

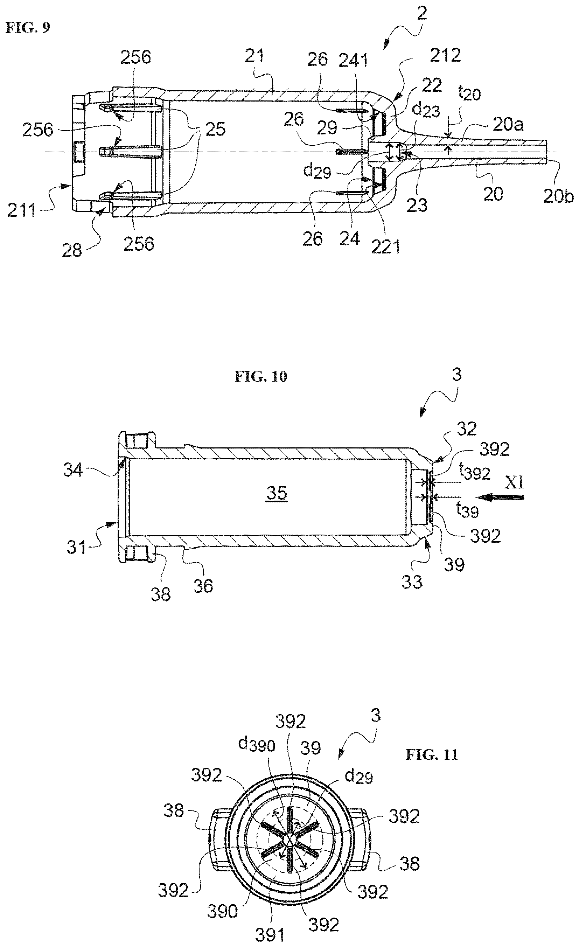

The sleeve 2 comprises a body 21 including a proximal open end 211 and a distal end 212 defined by a distal wall 22 of the sleeve. The distal wall 22 is provided with a dispensing hole 23, from which extends a tubular dispensing nozzle 20 formed in one piece with the body 21 of the sleeve. As can be seen in the longitudinal section of Figure 9, the thickness t₂₀ of the peripheral wall 20a of the dispensing nozzle 20 decreases from the dispensing hole 23 toward a free end 20b of the dispensing nozzle. Thanks to this specific geometry combined to the constitutive material of the dispensing nozzle 20, which is a polymer, preferably a transparent polymer such as a transparent amorphous copolyester in this example, the dispensing nozzle 20 is deformable while being capable of retaining a constant internal diameter even when it is bent. In this way, the dispensing nozzle 20 is steerable while having a constant diameter for the dispensing of material.

The distal wall 22 of the sleeve 2 comprises an internal piercing element 29 in the alignment of the dispensing hole 23, which is intended to break a breakable distal wall 39 of the barrel 3. The piercing element 29 is a tubular element projecting internally from the distal wall 22 of the sleeve so as to be in fluid communication with the dispensing hole 23. Thanks to this arrangement, once the distal wall 39 of the barrel 3 has been broken, the tubular piercing element 29 forms a privileged passageway guiding the material contained in the barrel 3 directly to the dispensing hole 23, which limits the losses of material in the interspace between the barrel 3 and the sleeve 2.

As shown in Figure 9, the inner diameter d₂₉ of the piercing element 29 is slightly higher than the diameter d₂₃ of the dispensing hole 23. In this way, the opening created in the distal wall 39 of the barrel 3 under the action of the piercing element 29 has a size suitable to ensure a proper flow of the material to be dispensed. Around the piercing element 29, the sleeve 2 comprises an annular (or ring-shaped) cavity 24 whose bottom is formed by the inner surface 221 of the distal wall 22, while the central wall of the ring is formed by the peripheral wall of the tubular piercing element 29 and the external wall of the ring is formed by a beveled inner surface 241 complementary to a corresponding beveled outer surface 33 of the barrel 3. The complementary beveled surfaces 241, 33 are designed to provide an efficient sealing at the interface between the sleeve 2 and the barrel 3 adjacent to the piercing element 29 and the dispensing hole 23, so as to limit the passage of material in the interspace between the barrel 3 and the sleeve 2.

As visible in Figures 10 and 11, the barrel 3 of the cartridge 1 comprises a proximal open end 31 and a distal end 32 defined by the breakable distal wall 39. The barrel 3 delimits a chamber 35 configured to receive the powder component of the two-component dental restoration material. The constitutive material of the barrel 3 is selected to have a Water Vapor Transfer Rate (WVTR) at 23°C and 85% RH, for a film thickness of 100 µm, of less than 0.5 g/(m²-day), so as to maintain a constant humidity level in the chamber 35 suitable to protect the moisture sensitive powder component. In particular, in this example, the barrel 3 is made of a cyclic olefin copolymer (COC) having a WVTR at 23°C and 85% RH, for a film thickness of 100 µm, of the order of 0.4 g/(m²-day). According to another embodiment, the constitutive material of the barrel 3 is selected to have a Water Vapor Transfer Rate (WVTR) at 23°C and 85% RH, for a film thickness of 100 µm, of higher than 0.5 g/(m²-day).

As clearly visible in Figure 11, the distal wall 39 of the barrel 3 comprises a central breakable portion 390 surrounded by a sealing portion 391 configured to cooperate with the inner surface 221 of the distal wall 22 of the sleeve. Advantageously, the ratio of the surface area of the breakable portion 390 to the surface area of the sealing portion 391 is such that, after opening, the fins of the open breakable portion 390 leave the dispensing hole 23 of the sleeve completely cleared, while the bearing surface of the sealing portion 391 is a sufficiently thin ring to limit the contact area. In this way, the seal is easier to achieve with the distal wall 22 of the sleeve and the contact pressure is maximized to ensure sealing during the dispensing of the material. This arrangement ensures a proper flow of the material to be dispensed while limiting the material losses.

The diameters of the breakable portion 390 and the piercing element 29 are also adjusted to maximize the breakage efficiency of the piercing element 29. For example, in the illustrated example, the diameter d₂₉ of the piercing element 29 is of the order of half the diameter d₃₉₀ of the breakable portion 390. As shown in Figure 11, the breakable portion 390 comprises six weakened lines 392 distributed radially in a star shape, starting from a central portion of the distal wall 39. Each weakened line 392 has a thickness t₃₉₂ lower than the thickness t₃₉ of the rest of the distal wall 39. The central portion of the star makes it possible to have a stress concentration so that the breaking starts in the center of the star.

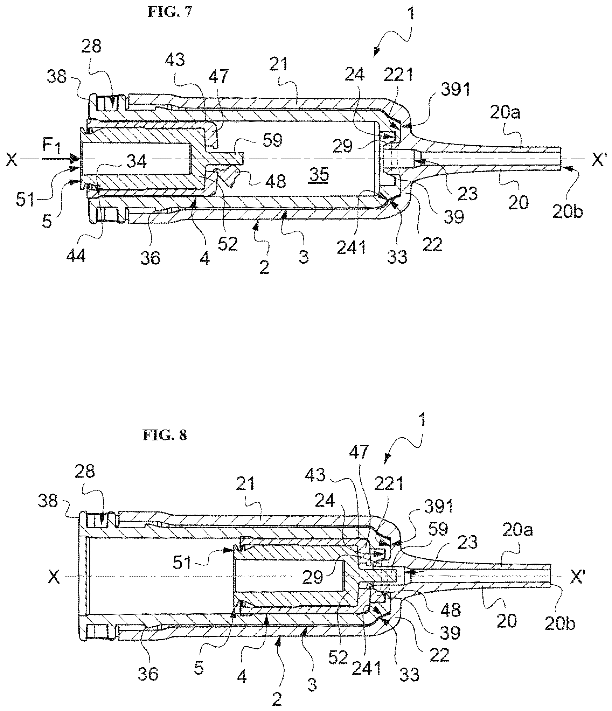

This arrangement of the weakened lines 392 is configured to guide a deformation of the distal wall 39 of the barrel, when it is submitted to the action of the piercing element 29, in a corolla shape oriented away from the dispensing nozzle 20. In practice, the rupture of the distal wall 39 is obtained through a displacement of the barrel 3 in the sleeve 2 along the longitudinal axis X-X' between a first position, visible in Figures 2, 4, 5, in which the distal wall 39 of the barrel is at a distance ei from the distal wall 22 of the sleeve, and a second position, visible in Figures 7, 8, in which the sealing portion 391 of the distal wall 39 of the barrel is in contact with the inner surface 221 of the distal wall 22 of the sleeve and the complementary beveled surfaces 241, 33 cooperate, thus providing a sealing between the sleeve 2 and the barrel 3.

The barrel 3 also comprises two diametrically opposed tabs 38 near its proximal end 31. Each tab 38 is configured to be received in a corresponding housing 278 defined by a locking wing 27 of the sleeve 2 arranged near the proximal end 21, so as to lock the barrel 3 in the first position with respect to the sleeve 2. This locked configuration of the cartridge 1 ensures that the distal wall 39 of the barrel 3 remains at the distance ei from the distal wall 22 of the sleeve, so that the cartridge 1 can be manipulated without any risk of the distal wall 39 being pierced by the piercing element 29, which makes it possible to implement a mixing step prior to the dispensing step.

In addition, the barrel 3 comprises an outer radial collar 36 configured to cooperate with inner radial recesses 256 formed by six clipping members 25 distributed circumferentially inside the body 21 of the sleeve 2. The cooperation between the outer radial collar 36 and the inner radial recesses 256 makes it possible to keep the barrel 3 in the first position with respect to the sleeve 2 even when the tabs 38 are disengaged from the housings 278, thus allowing the cartridge 1 to be secured in a storage configuration even when the locking system 38/278 is not active.

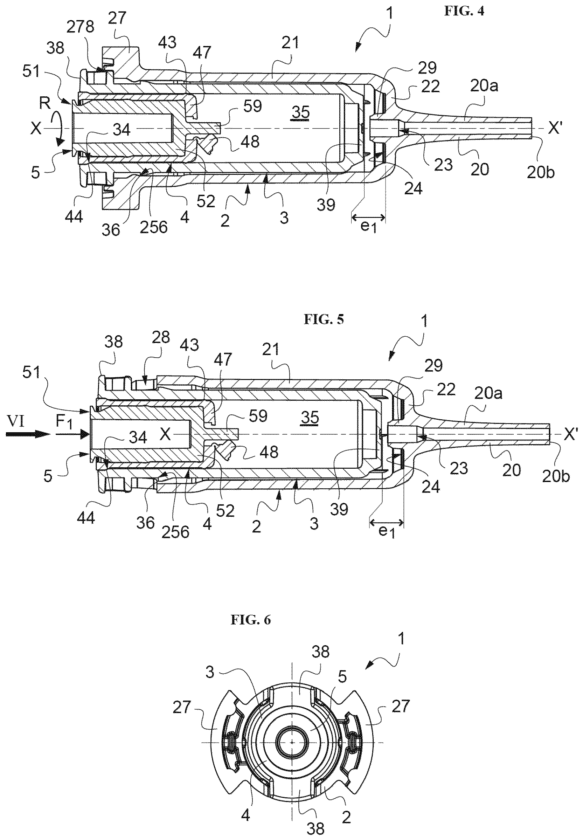

As can be seen particularly in Figure 5, the body 21 of the sleeve 2 also comprises two diametrically opposed longitudinal grooves 28 near its proximal end 211, which are configured to guide the displacement of the tabs 38 of the barrel 3 when the barrel is moved from the first position to the second position. The longitudinal grooves 28 ensure a guiding of the barrel 3 near the proximal end 211, in its movement towards the piercing element 29, while another guiding is ensured near the proximal end 212 by six guiding ribs 26 distributed circumferentially inside the body 21 of the sleeve. The guiding means 26 and 28 improve the stability of the displacement of the barrel 3 to reach the dispensing configuration of the cartridge 1.

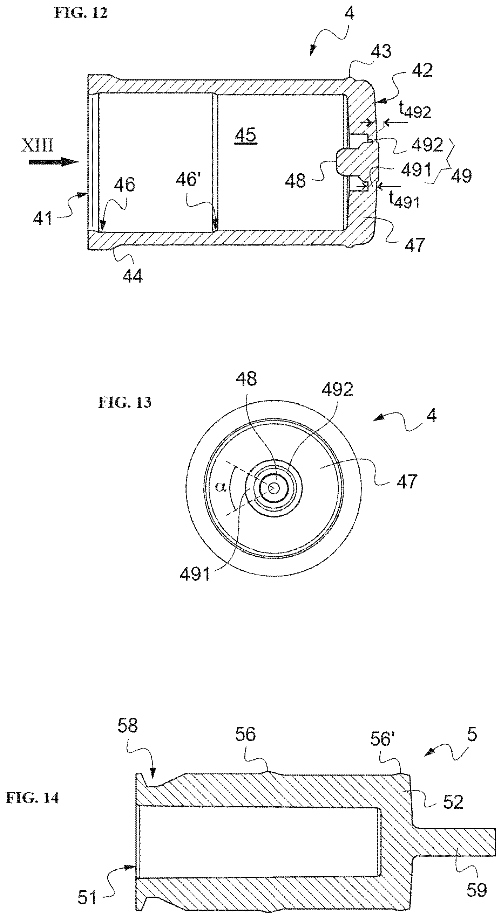

The chamber 35 of the barrel 3 is sealed by means of a piston formed by the combination of the liquid receptacle 4 and the plunger 5. In this illustrative example, the receptacle 4 is made of low-density polyethylene (LDPE) and the plunger 5 is made of acrylonitrile butadiene styrene (ABS). As shown in Figure 12, the receptacle 4 comprises a proximal open end 41 and a distal end 42 defined by an openable distal wall 47. The receptacle 4 delimits a chamber 45 configured to receive the liquid component of the two-component dental restoration material. The distal wall 47 of the receptacle 4 comprises a detachable portion 48 attached to the rest of the distal wall by an annular connecting part 49. As best seen in Figure 13, the annular connecting part 49 comprises a solid portion 491 extending over an angle α of the order of 60°, and a weakened portion 492 having a thickness t₄₉₂ less than the thickness t₄₉₁ of the solid portion 491, so as to form a hinge. This arrangement makes it possible to open the distal wall 47 of the receptacle 4 by rupturing only the weakened portion 492, so that the detachable portion 48 flips out of the receptacle 4 while remaining connected to the distal wall 47 of the receptacle 4 through the hinge formed by the solid portion 491.

The receptacle 4 is configured to move in sealing engagement in the chamber 35 of the barrel 3. To this end, the receptacle 4 is provided externally with a peripheral sealing rib 43 configured to prevent the passage of the liquid component, initially contained in the chamber 45 of the receptacle 4, toward the interspace between the barrel 3 and the receptacle 4, when the distal wall 47 of the receptacle is open and the receptacle 4 is moved towards the distal wall 39 of the barrel.

In practice, the opening of the distal wall 47 of the receptacle 4 is obtained thanks to a rod 59 extending distally from the distal wall 52 of the plunger 5. More precisely, the chamber 45 of the receptacle 4 is sealed by means of the plunger 5, which is movable in the chamber 45 and whose distal rod 59 is configured to apply a pressure on the detachable portion 48 of the distal wall 47 of the receptacle. Before the opening of the distal wall 47, the powder component is hermetically received in the chamber 35 of the barrel 5 and the liquid component is hermetically received in the chamber 45 of the receptacle 4. The two components can then be easily mixed together at a desired time, so as to form the material to be dispensed, by opening the openable distal wall 47 of the receptacle through application of a pressure on the detachable portion 48 with the distal rod 59 of the plunger and applying a vibration to the cartridge 1 in this activated configuration, either manually or by means of a vibration mixer.

The receptacle 4 comprises two inner peripheral grooves 46, 46' configured to cooperate with two corresponding outer peripheral ribs 56, 56' of the plunger 5 to keep the plunger 5 at a distance e₂ from the openable distal wall 47 of the receptacle 4 in a storage configuration of the cartridge 1. The cooperation between the inner peripheral grooves 46, 46' and the outer peripheral ribs 56, 56' is releasable under the effect of a thrust force F₀ applied on the proximal end 51 of the plunger 5, e.g. by means of an activation machine. The cooperating radial features 46, 56 and 46', 56' between the receptacle 4 and the plunger 5 make it possible to secure the cartridge 1 in the storage configuration where the powder component and the liquid component are separated, the first one being received in the chamber 35 of the barrel while the second one is received in the chamber 45 of the receptacle, without risk of accidental mixing of the two components.

The barrel 3 also comprises an inner peripheral groove 34 near its proximal end 31, which is configured to cooperate with an outer peripheral rib 44 of the receptacle 4 to keep the distal wall 47 of the receptacle 4 at a distance from the distal wall 39 of the barrel 3 before the piston formed by the combination of the receptacle 4 and the plunger 5 is moved towards the distal wall 39 of the barrel for the dispensing of the material. The cooperating radial features 34, 44 between the barrel 3 and the receptacle 4 make it possible to have a two-step transition of the cartridge 1 towards the dispensing configuration, obtained through the application of a thrust force Fi on the proximal end 51 of the plunger 5, e.g. by means of a dispensing apparatus.

More specifically, in a first step of initiating the dispensing of the material, the barrel 3 is displaced relative to the sleeve 2 from the first position to the second position, until the distal wall 39 of the barrel is in contact with the distal wall 22 of the sleeve and broken by the piercing element 29. In this first step, the radial features 34, 44 between the barrel 3 and the receptacle 4 are mutually engaged so that the barrel 3, the receptacle 4 and the plunger 5 move integrally with one another. In a second step of dispensing the material through the dispensing nozzle, the piston formed by the combination of the receptacle 4 and the plunger 5 is displaced relative to the barrel 3, the radial features 34, 44 between the barrel 3 and the receptacle 4 being mutually disengaged in this second step.

A method of dispensing a two-component material by means of the cartridge 1 according to the invention, where the two-component material comprises a powder component, initially received in the chamber 35 of the barrel 3, and a liquid component, initially received in the chamber 45 of the receptacle 4, comprises steps as described below.

Initially, the cartridge 1 is in the storage configuration shown in Figures 2 and 3, in which the plunger 5 is kept at a distance e₂ from the openable distal wall 47 of the receptacle 4 by cooperation between the inner grooves 46, 46' of the receptacle 4 and the outer ribs 56, 56' of the plunger 5. In this storage configuration, the powder component is hermetically received in the chamber 35 of the barrel 5 and the liquid component is hermetically received in the chamber 45 of the receptacle 4. Starting from this configuration, the assembly comprising the barrel 3, the receptacle 4 and the plunger 5 united in rotation, is turned relative to the sleeve 2 so as to insert the tabs 38 of the barrel in the housings 278 of the locking wings 27 of the sleeve, thus reaching the locked configuration shown in Figures 2 and 3, in which the distal wall 39 of the barrel 3 is kept at a distance ei from the distal wall 22 of the sleeve 2.

Then, a thrust force F₀ is applied on the proximal end 51 of the plunger 5, e.g. by means of an activation machine, so that the distal rod 59 of the plunger 5 applies a pressure on the detachable portion 48 of the distal wall 47 of the receptacle 4, thus reaching the activated configuration shown in Figure 4. The two components are then mixed together, so as to form the material to be dispensed, by applying a vibration to the cartridge 1, either manually or by means of a vibration mixer. Advantageously, in the activated configuration shown in Figure 4, the cartridge 1 can be vibrated without any risk of the distal wall 39 of the barrel 3 being pierced by the piercing element 29, thanks to the cooperation between the tabs 38 and the locking wings 27.

Once the two components have been mixed together, the assembly comprising the barrel 3, the receptacle 4 and the plunger 5, united in rotation, is turned relative to the sleeve 2 in the direction of the arrow R of Figure 4 so as to extract the tabs 38 of the barrel from the housings 278 of the sleeve, thus reaching the unlocked configuration shown in Figure 5. In this unlocked configuration, a thrust force Fi is applied on the proximal end 51 of the plunger 5, e.g. by means of a dispensing apparatus. In a first step of initiating the dispensing of the material, under the action of the thrust force Fi, the barrel 3 is displaced relative to the sleeve 2 from the first position to the second position, until the distal wall 39 of the barrel is in contact with the distal wall 22 of the sleeve and broken by the piercing element 29 as shown in Figure 7. In this first step, the barrel 3, the receptacle 4 and the plunger 5 move integrally with one another thanks to the cooperation of the radial features 34, 44.

In a second step of dispensing the material through the dispensing nozzle, under the action of the thrust force Fi, the radial features 34, 44 between the barrel 3 and the receptacle 4 are mutually disengaged, and the piston formed by the combination of the receptacle 4 and the plunger 5 is displaced relative to the barrel 3. The transition from the first step to the second step happens automatically under the action of the thrust force Fi, the cooperation between the radial features 34, 44 being released once the distal wall 39 of the barrel 3 is in contact with the distal wall 22 of the sleeve 2. In the configuration of end of distribution of the material, visible in Figure 8, the distal rod 59 of the plunger 5 is received in the internal volume of the piercing element 29, which allows to push the material to be dispensed as far as possible towards the dispensing hole 23 and the dispensing nozzle 20, thus limiting the losses of material.

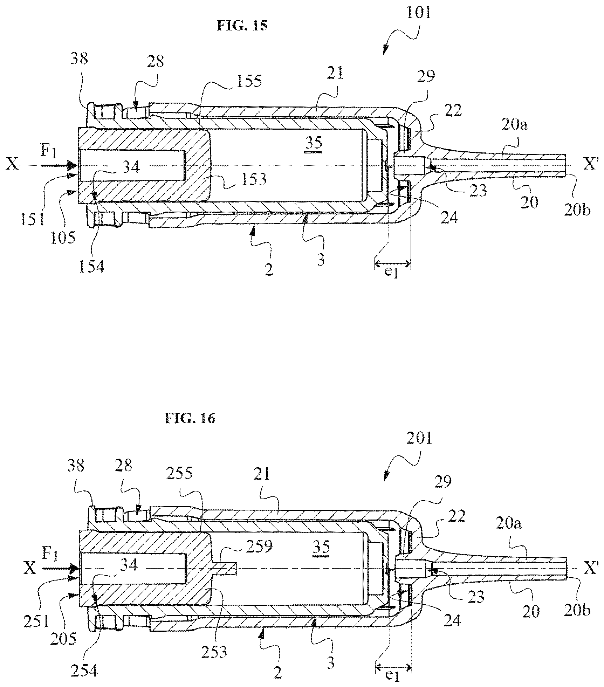

In the second embodiment shown in Figure 15, elements similar to those of the first embodiment bear identical references. The cartridge 101 of the second embodiment differs from the first embodiment in that it is suitable for the dispensing of a one-component material, the two-part piston 4, 5 of the first embodiment being replaced by a monobloc piston 105. In this second embodiment, the piston 105 comprises a distal plate 153 and is configured to move in sealing engagement in the chamber 35 of the barrel 3. In particular, the sealing is ensured by a peripheral sealing rib 155 of the piston 105. The inner peripheral groove 34 of the barrel 3 is configured to cooperate with an outer peripheral rib 154 of the piston 105 to keep the distal plate 153 at a distance from the distal wall 39 of the barrel 3 before the piston 105 is moved towards the distal wall 39 for the dispensing of the material. The cooperation between the groove 34 of the barrel 3 and the rib 154 of the piston 105 is released through the application of a thrust force Fi on the proximal end 151 of the piston 105, e.g. by means of a dispensing apparatus.

In the third embodiment shown in Figure 16, elements similar to those of the first embodiment bear identical references. The cartridge 201 of the third embodiment differs from the second embodiment only in that the piston 205 comprises a distal rod 259 extending distally from the distal plate 253. The distal rod 259 is configured to enter the tubular piercing element 29 of the sleeve 2 so as to push the material to be dispensed as far as possible towards the dispensing hole 23 and the dispensing nozzle 20, thus limiting the losses of material. Otherwise, the piston 205 is identical to the piston 105 of the second embodiment. In particular, the piston 205 comprises a peripheral sealing rib 255 ensuring that the piston 205 moves in sealing engagement in the chamber 35 of the barrel 3. The piston 205 also comprises an outer peripheral rib 254 configured to cooperate with the inner peripheral groove 34 of the barrel 3, their cooperation being released through the application of a thrust force Fi on the proximal end 251 of the plunger 205, e.g. by means of a dispensing apparatus.

A method of dispensing a material by means of the cartridge 101 or 201 is analogous to the method described above for the cartridge 1, only omitting the steps of mixing two components. Indeed, in the second and third embodiments, the material to be dispensed, which is received in the chamber 35 of the barrel 3, is ready to use. Initially, the cartridge 101 or 201 is in a configuration shown in Figures 15 and 16, in which the distal wall 39 of the barrel 3 is kept at a distance ei from the distal wall 22 of the sleeve 2. This configuration can be locked and unlocked, in a way similar to the first embodiment, by turning the assembly comprising the barrel 3 and the piston 105 or 205, united in rotation, relative to the sleeve 2 so as to insert or extract the tabs 38 of the barrel with respect to housings of the locking wings 27 of the sleeve. For the dispensing of the material, starting from the unlocked configuration shown in Figures 15 and 16, a thrust force Fi is applied on the proximal end 151 or 251 of the piston 105 or 205, e.g. by means of a dispensing apparatus.

In a first step of initiating the dispensing of the material, under the action of the thrust force Fi, the barrel 3 is displaced relative to the sleeve 2 from the first position to the second position, until the distal wall 39 of the barrel is in contact with the distal wall 22 of the sleeve and broken by the piercing element 29. In this first step, the barrel 3 and the piston 105 or 205 move integrally with one another thanks to the cooperation of the radial features 34 and 154, 254. In a second step of dispensing the material through the dispensing nozzle, under the action of the thrust force Fi, the radial features 34 and 154, 254 are mutually disengaged, and the piston 105 or 205 is displaced relative to the barrel 3. The transition from the first step to the second step happens automatically under the action of the thrust force Fi, the cooperation between the radial features 34 and 154, 254 being released once the distal wall 39 of the barrel 3 is in contact with the distal wall 22 of the sleeve 2.

The invention is not limited to the examples described and shown. In particular, other materials and shapes than those described above can be considered for the constitutive members of a cartridge according to the invention.

## Claims

1. Cartridge (1; 101; 201) for dispensing a material, comprising:
- a sleeve (2) having a longitudinal axis (X-X') and comprising a distal wall (22) provided with a dispensing hole (23);
- a barrel (3) defining a chamber (35) configured to receive the material, the barrel (3) comprising a proximal open end (31) and a breakable distal wall (39) at its distal end (32), the barrel (3) being configured to move in the sleeve (2) along the longitudinal axis (X-X') between a first position, in which the distal wall (39) of the barrel (3) is at a distance (e₁) from the distal wall (22) of the sleeve (2), and a second position, in which a sealing portion (391) of the distal wall (39) of the barrel (3) is in contact with an inner surface (221) of the distal wall (22) of the sleeve (2);
- a piston (4, 5; 105; 205) configured to seal the chamber (35) of the barrel (3), the piston (4, 5; 105; 205) comprising a plate (47; 153; 253) configured to move in sealing engagement in the chamber (35) of the barrel (3);
- wherein the sleeve (2) comprises an internal piercing element (29) in the vicinity of the dispensing hole (23), the piercing element (29) being configured to break the distal wall (39) of the barrel (3) upon transition of the barrel (3) from the first position to the second position; **characterised in that**
- the sleeve (2) comprises an annular cavity (24) around the piercing element (29), the annular cavity (24) being delimited peripherally by a beveled inner surface (241) complementary to a corresponding beveled outer surface (33) of the barrel (3).

2. Cartridge according to claim **1**, wherein the piercing element (29) is a tubular element projecting internally from the distal wall (22) of the sleeve (2), said piercing element (29) being in fluid communication with the dispensing hole (23).

3. Cartridge according to claim **2**, wherein the inner diameter (d₂₉) of the piercing element (29) is higher than or equal to the diameter (d₂₃) of the dispensing hole (23).

4. Cartridge according to any one of the preceding claims, wherein the distal wall (39) of the barrel (3) comprises at least one weakened portion (392) having a thickness (t₃₉₂) lower than the thickness (t₃₉) of the rest of the distal wall (39).

5. Cartridge according to any one of the preceding claims, wherein the barrel (3) is made of a material having a Water Vapor Transfer Rate (WVTR) at 23°C and 85% RH, for a film thickness of 100 µm, of less than 0.5 g/(m²-day), preferably less than 0.4 g/(m²-day).

6. Cartridge according to any one of the preceding claims, wherein the barrel (3) comprises at least one tab (38) near its proximal end (31) configured to cooperate with a corresponding housing (278) of the sleeve (2) to lock the barrel (3) in the first position with respect to the sleeve (2).

7. Cartridge according to claim **6**, wherein the barrel (3) comprises at least one outer radial feature (36) configured to cooperate with at least one inner radial feature (256) of the sleeve (2) to keep the barrel (3) in the first position with respect to the sleeve (2), even when the or each tab (38) of the barrel is disengaged from the corresponding housing (278) of the sleeve.

8. Cartridge according to any one of the preceding claims, wherein the sleeve (2) comprises at least one longitudinal groove (28) near the proximal end (21) configured to guide the displacement of a tab (38) of the barrel (3) when the barrel (3) is moved from the first position to the second position.

9. Cartridge according to any one of the preceding claims, wherein the barrel (3) comprises at least one inner radial feature (34) configured to cooperate with at least one outer radial feature (44; 154; 254) of the piston (4, 5; 105; 205) to keep the plate (47; 153; 253) of the piston at a distance from the distal wall (39) of the barrel (3) before the piston is moved towards the distal wall (39) of the barrel (3) for the dispensing of the material.

10. Cartridge according to any one of the preceding claims, comprising a deformable dispensing nozzle (20) connected to the dispensing hole (23), wherein the thickness (t₂₀) of the wall (20a) of the dispensing nozzle (20) decreases from the dispensing hole (23) toward a free end (20b) of the dispensing nozzle (20).

11. Cartridge according to any one of the preceding claims, wherein the piston (4, 5; 105; 205) comprises a distal rod (59; 259) configured to be received in an internal volume of the piercing element (29) when the cartridge (1) is in a configuration of end of distribution of the material.

12. Cartridge according to any one of the preceding claims, wherein the piston comprises a combination of a receptacle (4) and a plunger (5), the receptacle (4) defining a chamber (45) and comprising a proximal open end (41) and an openable distal wall (47), the plunger (5) being movable in the chamber (45) of the receptacle (4) and comprising a distal rod (59) configured to apply a pressure on the openable distal wall (47).

13. Cartridge according to claim **12**, wherein the openable distal wall (47) of the receptacle (4) comprises a detachable portion (48) attached to the rest of the distal wall (47) by an annular connecting part (49) having a solid portion (491) extending over an angle (α) of between 30° and 90°, preferably of the order of 60°, and a weakened portion (492) having a thickness (t₄₉₂) less than the thickness (t₄₉₁) of the solid portion (491), so as to form a hinge.

14. Cartridge according to claim **12** or claim **13**, wherein the receptacle (4) comprises at least one inner radial feature (46, 46') configured to cooperate with at least one outer radial feature (56, 56') of the plunger (5) to keep the plunger (5) at a distance (e₂) from the openable distal wall (47) of the receptacle (4) in a storage configuration of the cartridge (1), the cooperation between the inner radial feature (46, 46') and the outer radial feature (56, 56') of the plunger (5) being releasable under the effect of a thrust force (F₀) applied on the proximal end (51) of the plunger (5).

## Patentansprüche

1. Kartusche (1; 101; 201) zum Ausgeben eines Materials, umfassend:
- eine Hülse (2), die eine Längsachse (X-X') aufweist und eine distale Wand (22) umfasst, die mit einem Ausgabeloch (23) versehen ist;
- einen Zylinder (3), der eine Kammer (35) definiert, die konfiguriert ist, um das Material aufzunehmen, wobei der Zylinder (3) ein proximales offenes Ende (31) und eine zerbrechliche distale Wand (39) an seinem distalen Ende (32) umfasst, wobei der Zylinder (3) konfiguriert ist, um sich in der Hülse (2) entlang der Längsachse (X-X') zwischen einer ersten Position, in der die distale Wand (39) des Zylinders (3) in einem Abstand (e₁) von der distalen Wand (22) der Hülse (2) liegt, und einer zweiten Position, in der ein Dichtungsabschnitt (391) der distalen Wand (39) des Zylinders (3) in Kontakt mit einer Innenfläche (221) der distalen Wand (22) der Hülse (2) steht, zu bewegen;
- einen Kolben (4, 5; 105; 205), der konfiguriert ist, um die Kammer (35) des Zylinders (3) abzudichten, wobei der Kolben (4, 5; 105; 205) eine Platte (47; 153; 253) umfasst, die konfiguriert ist, sich in abdichtendem Eingriff in der Kammer (35) des Zylinders (3) zu bewegen;
- wobei die Hülse (2) ein inneres Durchstechelement (29) in der Nähe des Ausgabelochs (23) umfasst, wobei das Durchstechelement (29) konfiguriert ist, um die distale Wand (39) des Zylinders (3) beim Übergang des Zylinders (3) von der ersten Position in die zweite Position zu brechen; **dadurch gekennzeichnet, dass**
- die Hülse (2) einen ringförmigen Hohlraum (24) um das Durchstechelement (29) herum umfasst, wobei der ringförmige Hohlraum (24) peripher durch eine abgeschrägte Innenfläche (241) begrenzt ist, die komplementär zu einer entsprechenden abgeschrägten Außenfläche (33) des Zylinders (3) ist.

2. Kartusche nach Anspruch **1**, wobei das Durchstechelement (29) ein rohrförmiges Element ist, das von der distalen Wand (22) der Hülse (2) nach innen vorsteht, wobei das Durchstechelement (29) in Fluidverbindung mit dem Ausgabeloch (23) steht.

3. Kartusche nach Anspruch **2**, wobei der Innendurchmesser (d₂₉) des Durchstechelements (29) größer oder gleich dem Durchmesser (d₂₃) des Ausgabelochs (23) ist.

4. Kartusche nach einem der vorhergehenden Ansprüche, wobei die distale Wand (39) des Zylinders (3) mindestens einen geschwächten Abschnitt (392) umfasst, die eine Dicke (t₃₉₂) aufweist, die geringer ist als die Dicke (t₃₉) des Rests der distalen Wand (39).

5. Kartusche nach einem der vorhergehenden Ansprüche, wobei der Zylinder (3) aus einem Material mit einer Wasserdampfübertragungsrate (WVTR) bei 23 °C und 85 % relativer Luftfeuchtigkeit für eine Filmdicke von 100 µm oder weniger als 0,5 g/(m²-Tag), vorzugsweise weniger als 0,4 g/(m²-Tag), hergestellt ist.

6. Kartusche nach einem der vorhergehenden Ansprüche, wobei der Zylinder (3) mindestens eine Lasche (38) nahe seinem proximalen Ende (31) umfasst, die konfiguriert ist, um mit einem entsprechenden Gehäuse (278) der Hülse (2) zusammenzuwirken, um den Zylinder (3) in Bezug auf die Hülse (2) in der ersten Position zu verriegeln.

7. Kartusche nach Anspruch **6,** wobei der Zylinder (3) mindestens ein äußeres radiales Merkmal (36) umfasst, das konfiguriert ist, um mit mindestens einem inneren radialen Merkmal (256) der Hülse (2) zusammenzuwirken, um den Zylinder (3) in Bezug auf die Hülse (2) in der ersten Position zu halten, selbst wenn die oder jede Lasche (38) des Zylinders von dem entsprechenden Gehäuse (278) der Hülse gelöst ist.

8. Kartusche nach einem der vorhergehenden Ansprüche, wobei die Hülse (2) mindestens eine Längsnut (28) nahe dem proximalen Ende (21) umfasst, die konfiguriert ist, um die Verschiebung einer Lasche (38) des Zylinders (3) zu führen, wenn der Zylinder (3) von der ersten Position in die zweite Position bewegt wird.

9. Kartusche nach einem der vorhergehenden Ansprüche, wobei der Zylinder (3) mindestens ein inneres radiales Merkmal (34) umfasst, das konfiguriert ist, um mit mindestens einem äußeren radialen Merkmal (44; 154; 254) des Kolbens (4, 5; 105; 205) zusammenzuwirken, um die Platte (47; 153; 253) des Kolbens in einem Abstand von der distalen Wand (39) des Zylinders (3) zu halten, bevor der Kolben in Richtung der distalen Wand (39) des Zylinders (3) für das Ausgeben des Materials bewegt wird.

10. Kartusche nach einem der vorhergehenden Ansprüche, umfassend eine verformbare Ausgabedüse (20), die mit dem Ausgabeloch (23) verbunden ist, wobei die Dicke (t₂₀) der Wand (20a) der Ausgabedüse (20) von dem Ausgabeloch (23) in Richtung eines freien Endes (20b) der Ausgabedüse (20) abnimmt.

11. Kartusche nach einem der vorhergehenden Ansprüche, wobei der Kolben (4, 5; 105; 205) eine distale Stange (59; 259) umfasst, die konfiguriert ist, um in einem Innenvolumen des Durchstechelements (29) aufgenommen zu werden, wenn die Kartusche (1) sich in einer Konfiguration am Ende der Verteilung des Materials befindet.

12. Kartusche nach einem der vorhergehenden Ansprüche, wobei der Kolben eine Kombination aus einen Behälter (4) und einen Schieber (5) umfasst, wobei der Behälter (4) eine Kammer (45) definiert und ein proximales offenes Ende (41) und eine zu öffnende distale Wand (47) umfasst, wobei der Schieber (5) in der Kammer (45) des Behälters (4) bewegbar ist und eine distale Stange (59) umfasst, die konfiguriert ist, um einen Druck auf die zu öffnende distale Wand (47) auszuüben.

13. Kartusche nach Anspruch **12,** wobei die zu öffnende distale Wand (47) des Behälters (4) einen lösbaren Abschnitt (48) umfasst, der am Rest der distalen Wand (47) durch ein ringförmiges Verbindungsteil (49) befestigt ist, das einen festen Abschnitt (491), der sich über einen Winkel (α) zwischen 30° und 90°, vorzugsweise in der Größenordnung von 60°, erstreckt, und einen geschwächten Abschnitt (492) mit einer Dicke (t₄₉₂), die kleiner als die Dicke (t₄₉₁) des festen Abschnitts (491) ist, aufweist, um ein Scharnier zu bilden.

14. Kartusche nach Anspruch **12** oder Anspruch **13**, wobei der Behälter (4) mindestens ein inneres radiales Merkmal (46, 46') umfasst, das konfiguriert ist, um mit mindestens einem äußeren radialen Merkmal (56, 56') des Schiebers (5) zusammenzuwirken, um den Schieber (5) in einer Aufbewahrungskonfiguration der Kartusche (1) in einem Abstand (e₂) von der zu öffnenden distalen Wand (47) des Behälters (4) zu halten, wobei das Zusammenwirken zwischen dem inneren radialen Merkmal (46, 46') und dem äußeren radialen Merkmal (56, 56') des Schiebers (5) unter der Wirkung einer auf das proximale Ende (51) des Schiebers (5) aufgebrachten Schubkraft (F₀) lösbar ist.

## Revendications

1. Cartouche (1 ; 101 ; 201) pour distribuer un matériau, comprenant :
- un manchon (2) d'axe longitudinal (X-X') et comprenant une paroi distale (22) munie d'un trou de distribution (23) ;
- un cylindre (3) définissant une chambre (35) configurée pour recevoir le matériau, le cylindre (3) comprenant une extrémité ouverte proximale (31) et une paroi distale sécable (39) à son extrémité distale (32), le cylindre (3) étant configuré pour se déplacer dans le manchon (2) selon l'axe longitudinal (X-X') entre une première position, dans laquelle la paroi distale (39) du cylindre (3) est à une distance (e₁) de la paroi distale (22) du manchon (2), et une deuxième position, dans laquelle une partie d'étanchéité (391) de la paroi distale (39) du cylindre (3) est en contact avec une surface interne (221) de la paroi distale (22) du manchon (2) ;
- un piston (4, 5 ; 105 ; 205) configuré pour obturer la chambre (35) du cylindre (3), le piston (4, 5 ; 105 ; 205) comprenant une plaque (47 ; 153 ; 253) configurée pour se déplacer en prise étanche dans la chambre (35) du cylindre (3) ;
dans lequel le manchon (2) comprend un élément de perçage interne (29) au voisinage du trou de distribution (23), l'élément de perçage (29) étant configuré pour casser la paroi distale (39) du cylindre (3) lors de la transition du cylindre (3) de la première position à la seconde position ; **caractérisé en ce que**
- le manchon (2) comprend une cavité annulaire (24) autour de l'élément de perçage (29), la cavité annulaire (24) étant délimitée périphériquement par une surface intérieure biseautée (241) complémentaire d'une surface extérieure biseautée (33) correspondante du cylindre (3).

2. Cartouche selon la revendication **1**, dans laquelle l'élément de perçage (29) est un élément tubulaire faisant saillie intérieurement de la paroi distale (22) du manchon (2), ledit élément de perçage (29) étant en communication fluidique avec le trou de distribution (23).

3. Cartouche selon la revendication **2**, dans laquelle le diamètre intérieur (d₂₉) de l'élément de perçage (29) est supérieur ou égal au diamètre (d₂₃) du trou de distribution (23).

4. Cartouche selon l'une quelconque des revendications précédentes, dans laquelle la paroi distale (39) du cylindre (3) comprend au moins une partie affaiblie (392) ayant une épaisseur (t₃₉₂) inférieure à l'épaisseur (t₃₉) du reste de la paroi distale (39).

5. Cartouche selon l'une quelconque des revendications précédentes, dans laquelle le cylindre (3) est fait d'un matériau ayant un taux de transfert de vapeur d'eau (*« Water Vapor Transfert Rate* », WVTR) à 23°C et 85% HR, pour une épaisseur de film de 100 µm, inférieure à 0,5 g/(m²-jour), de préférence inférieure à 0,4 g/(m²-jour).

6. Cartouche selon l'une quelconque des revendications précédentes, dans laquelle le cylindre (3) comprend au moins une languette (38) à proximité de son extrémité proximale (31) configurée pour coopérer avec un logement correspondant (278) du manchon (2) pour verrouiller le cylindre (3) dans la première position par rapport au manchon (2).

7. Cartouche selon la revendication **6**, dans laquelle le cylindre (3) comprend au moins un élément radial externe (36) configuré pour coopérer avec au moins un élément radial interne (256) du manchon (2) pour maintenir le cylindre (3) dans la première position par rapport au manchon (2), même lorsque la ou chaque languette (38) du cylindre est dégagée du logement correspondant (278) du manchon.

8. Cartouche selon l'une quelconque des revendications précédentes, dans laquelle le manchon (2) comprend au moins une rainure longitudinale (28) à proximité de l'extrémité proximale (21) configurée pour guider le déplacement d'une languette (38) du cylindre (3) lorsque le cylindre (3) est déplacé de la première position à la deuxième position.

9. Cartouche selon l'une quelconque des revendications précédentes, dans laquelle le cylindre (3) comprend au moins un élément radial interne (34) configuré pour coopérer avec au moins un élément radial externe (44 ; 154 ; 254) du piston (4, 5 ; 105 ; 205) pour maintenir la plaque (47 ; 153 ; 253) du piston à distance de la paroi distale (39) du cylindre (3) avant que le piston ne soit déplacé vers la paroi distale (39) du cylindre (3) pour la distribution du matériau.

10. Cartouche selon l'une quelconque des revendications précédentes, comprenant une buse de distribution déformable (20) reliée au trou de distribution (23), dans laquelle l'épaisseur (t₂₀) de la paroi (20a) de la buse de distribution (20) diminue depuis le trou de distribution (23) vers une extrémité libre (20b) de la buse de distribution (20).

11. Cartouche selon l'une quelconque des revendications précédentes, dans laquelle le piston (4, 5 ; 105 ; 205) comprend une tige distale (59 ; 259) configurée pour être reçue dans un volume interne de l'élément de perçage (29) lorsque la cartouche (1) est dans une configuration de fin de distribution du matériau.

12. Cartouche selon l'une quelconque des revendications précédentes, dans laquelle le piston comprend une combinaison d'un réceptacle (4) et d'un piston plongeur (5), le réceptacle (4) définissant une chambre (45) et comprenant une extrémité proximale ouverte (41) et une paroi distale ouvrante (47), le piston plongeur (5) étant mobile dans la chambre (45) du réceptacle (4) et comprenant une tige distale (59) configurée pour appliquer une pression sur la paroi distale ouvrante (47).

13. Cartouche selon la revendication **12**, dans laquelle la paroi distale ouvrable (47) du réceptacle (4) comprend une partie détachable (48) fixée au reste de la paroi distale (47) par une pièce de liaison annulaire (49) ayant une partie pleine (491) s'étendant sur un angle (α) compris entre 30° et 90°, de préférence de l'ordre de 60°, et une partie affaiblie (492) ayant une épaisseur (t₄₉₂) inférieure à l'épaisseur (t₄₉₁) de la partie pleine (491), de manière à former une charnière.

14. Cartouche selon la revendication **12** ou la revendication **13**, dans laquelle le réceptacle (4) comprend au moins un élément radial interne (46, 46') configuré pour coopérer avec au moins un élément radial externe (56, 56') du piston plongeur (5) pour maintenir le piston plongeur (5) à distance (e₂) de la paroi distale ouvrante (47) du réceptacle (4) dans une configuration de stockage de la cartouche (1), la coopération entre l'élément radial interne (46, 46') et l'élément radial externe (56, 56') du piston plongeur (5) étant libérable sous l'effet d'une force de poussée (F₀) appliquée sur l'extrémité proximale (51) du piston plongeur (5).
